# EUROPÄISCHE PATENTSCHRIFT

(11) **EP 2 675 461 B1**
(45) Veröffentlichungstag und Bekanntmachung des Hinweises auf die Patenterteilung: **12.07.2017**
(21) Anmeldenummer: 12713578.8
(22) Anmeldetag: 27.01.2012
(51) Int. Cl.: A61K 31/7048, A61K 35/20, A61K 36/70, A23L 5/00, A23L 33/105, A61P 3/08, A61P 9/12, A61P 9/10, A61P 19/10, A61P 25/02, A61P 27/12, A61P 25/28, A61P 17/18

(54) **DIÄTETISCHE ODER PHARMAZEUTISCHE ZUBEREITUNG ENTHALTEND TARTARISCHEN BUCHWEIZEN**
DIETARY AND PHARMACEUTICAL COMPOSITION COMPRISING FAGOPYRUM TATARICUM
COMPOSITION DIÉTÉTIQUE ET PHARMACEUTIQUE CONTENANT DU FAGOPYRUM TATARICUM

(30) Priorität: 16.02.2011 DE 102011011402; 20.06.2011 DE 102011105406
(43) Veröffentlichungstag der Anmeldung: 25.12.2013
(73) Patentinhaber: Thanares GmbH, 14473 Potsdam (DE)
(72) Erfinder: THIEDE, Hans-Michael, 14473 Potsdam (DE); KEHR, Wolfgang, 14195 Berlin (DE)
(74) Vertreter: Jungblut, Bernhard Jakob
(86) Internationale Anmeldenummer: PCT/DE2012/000062
(87) Internationale Veröffentlichungsnummer: WO 2012/110017

(56) Entgegenhaltungen:
- WO-A1-00/15237
- F. J. ZELLER ET AL: "Buchweizen- die vergessene Kulturpflanze: Funktionelles Lebensmittel", BIOLOGIE IN UNSERER ZEIT, Bd. 34, Nr. 1, 1. Januar 2004 (2004-01-01) , Seiten 24-31, XP55031232, ISSN: 0045-205X, DOI: 10.1002/biuz.200410241 in der Anmeldung erwähnt
- DATABASE WPI Week 200252 Thomson Scientific, London, GB; AN 2002-483260 XP002679853, & JP 2002 101833 A (OKUYAMA H) 9. April 2002 (2002-04-09)
- DATABASE WPI Week 201075 Thomson Scientific, London, GB; AN 2010-L69645 XP002679858, & CN 101 810 276 A (ZHENG J) 25. August 2010 (2010-08-25)

## Beschreibung

### Gebiet der Erfindung

Die Erfindung betrifft ein Verfahren zur Herstellung einer Zubereitung enthaltend gemahlenen Buchweizen und Milch, eine mit diesem Verfahren erhältliche Zusammensetzung und Verwendungen derselben.

### Stand der Technik und Hintergrund der Erfindung

4-Methyl-Catechol (4MC) ist eine in sehr geringen Konzentrationen im menschlichen Organismus vorkommende endogene Verbindung, über deren Bildungsort und Regulation des Stoffwechsels im menschlichen Gewebe wenig bekannt ist. Bekannt ist allerdings, dass 4-MC als Stoffwechselprodukt von oral aufgenommenen Flavonoiden wie Quercetin und Rutin durch die Aktivität der intestinalen Mikrobiota im Dickdarm des Menschen entstehen kann.

In in-vitro Untersuchungen und in tierexperimentellen Studien sind Wirkungen von 4-MC beschrieben worden, die eine präventive und/oder therapeutische Wirkung beim Menschen nahelegen. Im Folgenden werden die verschiedenen Wirkungen und die daraus jeweils abgeleiteten therapeutischen Anwendungen beschrieben:
1. Antientzündliche Wirkungen
2. Peroxyl-Radikal und Superoxid Anion-Radikal Fänger
3. Stimulation der Neurotrophine "Nerve Growth Factor" (NGF), "Brain Derived Nerve Growth Factor" (BDNF), Glia Derived Nerve Growth Factor (GDNF) u.a.
4. Hemmung der Cholesterin-Biosynthese
5. Hemmung der Lipoxygenase
6. Stimulation der Häm-Oxygenase
7. Hemmung der "Angiotensin Converting Enzyme" (ACE)-Aktivität

Zwischen diesen verschiedenen Wirkungen sind bestimmte kaskadenartige Abhängigkeiten beschrieben worden. 4-MC stimuliert den Phosphoinositol-3-Kinase/AKT Signaltransduktionsweg und aktiviert dadurch die Expression der Häm-Oxygenase, die ihrerseits auch als Reaktion auf oxidativen Stress neben Eisen die Bildung von Bilirubin und Kohlenmonoxid erhöht, die wiederum die Expression der Neurotrophine BDNF und GDNF in Neuronen wie auch Gliazellen anregen.(Furukawa,Y. et al., Biomedical Research 2010, 31: 45-52; Hung, S.Y. et al., Neuropharmacology 2010 Feb., 58: 321-329)

Alle vorstehend beschriebenen Effekte sind hinsichtlich einer neuroprotektiven Wirkung bedeutsam und unterstreichen die Funktion und Bedeutung der intestinalen mikrobiellen Aktivität.

### Periphere und autonome Neuropathien:

Zu den Folgeerkrankungen des Diabetes mellitus gehört die diabetische Neuropathie, von der 30 - 50% der Diabetiker betroffen sind und die damit die häufigste periphere Neuropathie in den westlichen Ländern ist (Pittenger G, Vinik A., Exp Diabesity Res. 2003 Oct-Dec,4(4):277-85. Review). Als Erkrankung des peripheren Nervensystems betrifft sie sowohl sensible als auch motorische Nerven und durch die Heterogenität sind neben den stark myelinisierten auch die dünn myelinisierten Fasern betroffen. Eine gefürchtete, und für die Betroffenen stark beeinträchtigende Komplikation, ist der sog. "diabetische Fuß" (diabetische Podopathie). Sensibilitätsstörungen mit und ohne Durchblutungsstörungen entwickeln sich dabei zu einem klinischen Bild das häufig zu einer Amputation der unteren Extremität führt. Jahr für Jahr werden aufgrund dieser Diagnose allein in Deutschland etwa 30.000 Amputationen der unteren Extremität durchgeführt (Chantelau E., Deutsches Ärzteblatt 2002, 99: A 2052-2056).

Aus tierexperimentellen Untersuchungen an Streptozotozin behandelten, diabetischen Ratten ist bekannt, dass die Expression von Neurotrophinen (Nervenwachstumsfaktoren) wie "Glia Cell derived neurotrophic factor" (GDNF), Neurotrophin 3 und NGF im Darm reduziert ist. Dies wird zur Erklärung der bekannten gastrointestinalen Komplikationen bei Diabetes-Patienten herangezogen (Liu, W et al., Auton Neurosci. 2010, 154: 79-83)

Seit Anfang der neunziger Jahre ist bekannt, dass es eine Reihe von Verbindungen gibt, die in der Lage sind, die endogene Neurotrophinbildung zu stimulieren (Furukawa Y, et al., Biochem Pharmacol. 1990 Nov 75; 40(10):2337-42). Zu diesen Substanzen gehören die Alkylcatechole und hier insbesondere das 4-MC. Für 4-MC ist gezeigt worden, dass Neuropathien in diversen tierexperimentellen Modellen (Resiniferatoxin, Pyridoxin, Acrylamid und Cytostatika induzierte Neuropathien sowie auch die Streptozotozin induzierte diabetische Neuropathie) durch die Gabe von 4-MC günstig beeinflusst werden (Hanaoka Y, et al., J Neurol Sci. 1994 Mar; 122(1):28-32). Ebenso wird die Gentamycininduzierte Ototoxizität durch 4-MC via Stimulation von NGF und anderen Neurotrophinen antagonisiert.

Auch im "crush-injury" Modell des Ischiasnerven der Maus verbesserte 4-MC die Reinnervation der Hautnerven insbesondere der nicht-myelinisierten Nervenfasern (Hsieh, YL et al., J Neuropathol Exp Neurol. 2009; 68: 1269-128 7).

Diese Ergebnisse legen nahe, dass Alkylcatechole und ihre Derivate auch beim Menschen präventive und therapeutische Wirkungen bei verschiedenen Formen von Neuropathien haben, wie bei
- diabetischer Neuropathie aber auch bei
- Chemotherapie induzierter Neuropathie und
- Neuropathie in Folge von chronischem Alkoholabusus

### Zentralnervös degenerative Erkrankungen:

Allein in Deutschland leiden derzeit mehr als eine Million Menschen unter einer Demenzerkrankung, etwa 700.000 davon unter Morbus Alzheimer, einer neurodegenerativen Erkrankung. Jedes Jahr werden ca. 200.000 neue Demenzerkrankungen diagnostiziert, von denen etwa 120.000 vom Alzheimertyp sind. An M. Parkinson sind in Deutschland derzeit 300.000-400.000 erkrankt. Aufgrund der demographischen Entwicklung wird die Prävalenz beider Erkrankungen zunehmen. Die finanziellen Belastungen des Gesundheitssystems durch diese Erkrankungen sind sehr hoch - die Behandlungs- und Pflegekosten eines Alzheimer Patienten liegen heute bei etwa 40.000 € pro Jahr - und werden weiter steigen. Therapeutika zur symptomatischen Behandlung des M. Parkinson stehen heute zwar schon zur Verfügung und erste Produkte zur Verbesserung kognitiver Funktionen bei Patienten mit M. Alzheimer zeigen marginale Wirkung, ein wirklicher Durchbruch mit Wirkstoffen, die die Progredienz dieser neurodegenerativen Erkrankungen aufhalten, konnte allerdings trotz intensiver weltweiter Forschung bisher nicht erzielt werden.

Die Wirkungen der Alkylcatechole und ihrer Metabolite weisen auf ein deutliches therapeutisches Potential hin: Neben der schon erwähnten Stimulation der Neurotrophine, die auch im Zentralnervensystem stattfindet und dem Neurodegenerationsprozess entgegen wirkt, sind Signaltransduktionswirkungen beschrieben worden, die nahelegen, dass 4-MC durch Aktivierung der Häm-Oxigenase-1-Expression neuroprotektive Wirkungen besitzt insbesondere gegenüber dem schädlichen oxidativem Stress (Furukawa, Y. et al., Biomedical Res 2010; 31: 45-52). 4-MC stimuliert auch die Mitogen-aktivierte Proteinkinase (MAPK/ERK1/2), die ihrerseits das "cAMPresponse element binding protein" (CREB) aktiviert. CREB spielt eine wichtige Rolle sowohl für das Nervenwachstum als auch für das Überleben der Nervenzellen.

Oxidativer Stress ist assoziiert mit dem Untergang von Nervenzellen und spielt eine große Rolle in der Pathogenese vieler chronischer degenerativer Erkrankungen wie dem M. Alzheimer, M. Parkinson, der Huntington'schen Chorea und der amyotrophen Lateralsklerose. Ein Signaltransduktionsweg, bei dem die Transkriptionsaktivierung von protektiven Genen durch ein "cis-acting element", dem sogenannten "Antioxidant responsive element" (ARE) vermittelt wird, ist von zunehmender Bedeutung. Aktivierung durch den Transkriptionsfaktor NF-E2-related factor 2 (Nrf2), der an ARE bindet, schützt Nervenzellen vor oxidativem Stress induziertem Zelltod (Johnson, J.A., et al., Ann NY acad Sci. 2008; 1147: 61-69). 4-MC aktiviert Nrf2 und kann auch über diesen Signaltransduktionsweg neuroprotektiv wirken (Satoh T., et al. Biochem Biophys Res Commun. 2009; 379: 537-341).

Alkyl-Catechole wie 4-MC und/oder 3,4-Dihydroxyphenylessigsäure (ein weiterer Metabolit des Rutins) besitzen zusätzliche antientzündliche Eigenschaften, die sich äußern in der Hemmung der Expression der induzierbaren NO-Synthase sowie der Hemmung der Freisetzung von proinflammatorischen Zytokinen wie TNF aus Mikroglia, womit klare neuroprotective Wirkungen verbunden sind.(Zheng, L.T. et al., Eur J Phormacol. 2008; 588: 106-113). Diese Schutzwirkungen lassen sich zur Behandlung neurodegenerativer Erkrankungen nutzen, die mit ausgeprägter Aktivierung der Mikroglia assoziiert sind.

Die Aufrechterhaltung bzw Verbesserung kognitiver Funktionen ist für demente Patienten, z.B. Patienten mit Morbus Alzheimer, von großer Bedeutung. Hinweise, das Alkylcatechole einen positiven Einfluss auf kognitive Leistung haben, ergeben sich aus Untersuchungen von Sun, M.K. et al., Neuroreport. 2008; 19: 355-359) Nach intraventrikulärer Verabreichung verbesserte 4-MC auch das räumliche Lernen und Gedächtnis von Ratten, eine Wirkung, an der offenbar BDNF beteiligt ist, denn gleichzeitige Verabreichung von BDNF-Antikörpern hob die Wirkung von 4-MC auf.

Auch O-Methyl-Metabolite von Alkylcatecholen wie 2-Methoxy-4-ethylphenol haben neuroprotektive Wirkungen, die sich therapeutisch bei der Behandlung von degenerativen Erkrankungen des Zentralnervensystems nutzen lassen. Sie schützen Nervenzellen - wie an Hippocampus-Neuronen gezeigt wurde - vor dem durch NMDA-Rezeptoren vermittelten exzessiven, neurotoxischen Einstrom von Kalziumionen (Fukumori R. et al., J Pharmacol Sci. 2010; 112: 273-281).

### Bluthochdruck/Atherosklerose:

Aus in-vitro Untersuchungen an Leberzellen geht hervor, dass nicht nur Quercetin sondern auch 4-MC die hepatozelluläre Cholesterinsynthese im µMol-Bereich hemmt (Glässer G. et al., Phytomedicine. 2002; 9: 33-40) Hemmung des Angiotensin Converting Enzymes und anderer Metallopeptidasen ist beschrieben in Bormann H., et al., Pharmazie. 2000; 55: 129-132)

### Diabetes mellitus:

Hemmung der nicht-oxidativen AGE (Advanced Glycation Endproduct) Bildung durch 4-MC und DOPAC wurde gezeigt in Pashikanti,S. et al., Free Radic Biol Med. 2009 Dec.4

### Osteoporose:

Metabolite der Alkylcatechole wie 2-Methoxy-4-methylphenol (Creosol) und 2-Methoxy-4-ethylphenol, die durch die katalytische Wirkung der Catechol-O-methyltransferase entstehen, verhindern die nach Ovariektomie von Mäusen eintretende Osteoporose - einem experimentellen Modell der postmenopausalen Osteoporose - wahrscheinlich durch Hemmung der Knochen abbauenden Osteoklasten in Verbindung mit einer anti-oxidativen Wirkung auf die Knochenwachstum fördernden Osteoblasten (Moriguchi N., et al., Biochem Pharmacol 2007; 73: 385-393). Auch für Hydroxytyrosol (3,4-Dihydroxyphenyl-ethanol) ist eine entsprechende Knochen protektive Wirkung an ovariektomierten Ratten beschrieben worden (Puel C. et al. J Agric Food Chem. 2008; 56: 9417-9422).

Zubereitungen enthaltend gemahlenen Buchweizen und Milch sind als Lebensmittel aus der Praxis sowie der Literaturstelle JP 2008 271 811 A bekannt. Gemäß dieser Literaturstelle werden Buchweizenkörner zunächst einer thermischen Dampfbehandlung unterzogen. Die Milch ist vor der Beimischung des Buchweizens nicht erwärmt. Aus der Literaturstelle Zeller, F.J., et al., Biol. Unserer Zeit, 34. Jahrgang 2004, Nr. 1, Seiten 24 ff, ist es bekannt, normalen Buchweizen mit verdünnter und erwärmter Milch zu mischen.

### Technisches Problem der Erfindung

Der Erfindung liegt das technische Problem zu Grunde, eine diätetische Zusammensetzung sowie deren Herstellungsverfahren zur Verfügung zu stellen, welche hohe Mengen an 4-MC im Organismus zur Verfügung stellt.

### Grundzüge der Erfindung und bevorzugte Ausführungsformen

Zur Lösung dieses technischen Problems lehrt die Erfindung ein Verfahren nach Anspruch 1.

Der eingesetzte gemahlene tartarische Buchweizen braucht keiner Vorbehandlung unterzogen zu werden, insbesondere ist es entbehrlich, Buchweizenkörner einer Dampfbehandlung zu unterziehen.

Gegenüber der Mischung des tartarischen Buchweizens mit kalter Milch bzw. solcher mit Raumtemperatur ist die erfindungsgemäße Vorgehensweise insofern vorteilhaft, als dass eine Reaktion Rutin zu Quercetin nicht bzw. kaum stattfindet durch thermische Inaktivierung der entsprechenden Enzyme und ein bitterer Geschmack vermieden wird.

Die Erfindung beruht auf der Erkenntnis, dass überraschenderweise die Menge im Organismus verfügbaren 4-MC, gebildet im Wesentlichen aus im tartarischen Buchweizen enthaltenen Rutin, besonders hoch ist, wenn die Zubereitung mit aufgekochter heißer Proteinlösung oder unter Aufkochen der Mischung aus dem Buchweizen und der Proteinlösung hergestellt wird, im Vergleich mit Einsatz von Wasser oder kalter Proteinlösung und ohne Erhitzung.

Dem erfindungsgemäßen Verfahren liegt das Grundkonzept zu Grunde, in dem tartarischen Buchweizen enthaltene endogene Enzyme zu inaktivieren, so dass eine möglichst hohe Ausbeute an 4-MC erzielt werden kann.

In einer Weiterbildung des erfindungsgemäßen Verfahrens kann die Mischung in jeder beliebigen Verfahrensstufe, unabhängig von anderen Verfahrensstufen, entweder nicht agitiert oder agitiert, insbesondere gerührt, werden, für eine Dauer von 0,1-200 min., insbesondere 1-60 min. Der Begriff der Raumtemperatur, sofern nicht vorstehend oder folgend andere Bereiche als geeignet angegeben sind, bezieht sich auf eine Temperatur im Bereich von 15-25 °C, beispielsweise 20 °C.

Der Begriff der Proteinlösung bezeichnet Mischungen von Wasser und Proteinen, wobei die Proteine in der wässrigen Phase gelöst oder darin suspendiert sein können. Eine im Rahmen der Erfindung geeignete Proteinlösung weist in der Regel, aber nicht zwingend, einen Proteingehalt von 0,1 bis 20 Gew.-%, insbesondere 1 bis 5 Gew.-%, vorzugsweise 2 bis 4 Gew.-%, Protein auf, bezogen auf die Menge an eingesetzter Proteinlösung. Die Bestimmung des Proteingehaltes kann insbesondere mittels in der Lebensmittelindustrie, insbesondere der Milchindustrie, standardisierter Kjeldahl-Methoden gemessen werden, wobei als Faktor zur Bestimmung des Proteingehaltes aus dem Stickstoffgehalt einer Probe 6,25 angesetzt wird, i.e. der Gewichtsanteil Stickstoff wird mit 6,25 multipliziert und ergibt dann den Gewichtsanteil an Protein in der Proteinlösung.

Tartarischer Buchweizen wird auch als falscher Buchweizen bezeichnet. Die Artenbezeichnung lautet Fagopyrum tataricum. Eine Subspezies ist Fagopyrum tataricum ssp. potanini. Tartarischer Buchweizen zeichnet sich durch einen gegenüber anderen Buchweizenarten vergleichsweise sehr hohen Gehalt an Rutin aus (siehe auch Zeller, F.J., Die Bodenkultur, 52(3): 259 ff., (2001).

Der Begriff des gemahlenen Buchweizen umfasst als Mahlprodukte Mehl (Teilchengröße < 180 µm, gemessen und fraktioniert mit Sieben), Dunst (80 - 300 µm), Grieß (300 - 1000 µm) und Schrot (> 1000 µm), jeweils für sich oder in beliebiger Mischung miteinander. Im Rahmen dieser Beschreibung wird der Begriff "Buchweizen" insbesondere als Synonym für Buchweizensamen verwendet.

Das Gewichtsverhältnis Proteinlösung zu Buchweizen beträgt 4 - 20. Der Bereich des Gewichtsverhältnisses Proteinlösung zu Buchweizen von 4 bis 20 meint, dass ein Gewichtsteil Buchweizen als Untergrenze des Bereiches mit 4 Gewichtsteilen Proteinlösung gemischt werden, während die Obergrenze des Bereiches die Mischung von einem Gewichtsteil Buchweizen mit 20 Gewichtsteilen Proteinlösung ist.

Die Proteinlösung ist ausgewählt aus der Gruppe bestehend aus Kuhmilch, Buttermilch, Kamelmilch, Stutenmilch, Ziegenmilch, Eselsmilch, Lamamilch, Rentiermilch, Wasserbüffelmilch und Schafsmilch. Aber auch Proteinlösungen pflanzlichen Ursprungs sind einsetzbar, nämlich Sojamilch oder Kokosmilch. Die Proteinlösung kann auch aus einem Milchpulver, wobei das Pulver aus einer oder mehreren der vorstehenden genannten Milcharten gewonnen wurde, durch Mischung mit Wasser hergestellt werden. Für bestimmte Anwendungsbereiche, beispielsweise Menschen mit Laktose Intoleranz oder für Anwendung bei Tieren, kann vorgesehen sein, dass die Proteinlösung bzw. Milch Laktose-frei ist. Bevorzugt ist wegen der Verfügbarkeit und Wirtschaftlichkeit Kuhmilch. Ist die Proteinlösung eine Milch, so weist sie typischerweise einen Fettgehalt von 0,5 - 8%, insbesondere 1,3 - 6%, auf. Sie kann unbehandelt, pasteurisiert, einem Extended Shelf Life (ESL) Verfahren unterworfen, oder ultrahocherhitzt sein. Diesbezügliche Behandlungsverfahren sind dem Fachmann aus der Lebensmitteltechnologie gut bekannt und brauchen daher hier nicht näher beschrieben werden.

In einer bevorzugten Variante der Erfindung wird die Proteinlösung vor der Zugabe des Buchweizens auf eine Temperatur von zumindest 80 °C, vorzugsweise zumindest 90 °C, gebracht und bis zur Zugabe des Buchweizens auf dieser Temperatur gehalten.

In Stufe b) wird die Mischung auf jeden Fall auf eine Temperatur von vorzugsweise zumindest 90 °C, gebracht bzw. gehalten.

In Stufe c) wird die Mischung vorzugsweise auf eine Temperatur von 5 - 40 °C, insbesondere 8 - 30 °C, abgekühlt.

Die so erhaltene Zubereitung ist als solche zum unmittelbaren menschlichen oder tierischen Verzehr geeignet. Für weitere Anwendungen ist es jedoch bevorzugt, wenn anschließend an Stufe c) eine Trocknung, insbesondere eine Gefriertrocknung (Lyophilisation) der Zubereitung durchgeführt wird. Der Begriff der Trocknung bezeichnet den Entzug von Wasser bis zu einem Restwassergehalt von weniger als 5 Gew.-%, vorzugsweise weniger als 2 Gew.-%, bezogen auf das Gesamtgewicht der getrockneten Zubereitung. Grundsätzlich sind aber auch andere Trocknungsverfahren, beispielsweise die Sprühtrocknung, einsetzbar.

Es ist aber auch möglich, die Mischung üblichen lebensmitteltechnologischen Verfahren zur Weiterverarbeitung nach der jeweiligen abschließenden Verfahrensstufe der verschiedenen vorstehenden Varianten der Erfindung zu unterwerfen, beispielsweise der Weiterverarbeitung zu Jogurt, Kefir, Käse, oder Molke.

Die Erfindung betrifft auch eine diätetische oder pharmazeutische Zubereitung erhältlich mit einem der vorstehenden erfindungsgemäßen Verfahren. Sie enthält typischerweise 0,5 - 10 Gew.-%, insbesondere 1 - 5 Gew.-%, vorzugsweise 2 - 4 Gew.-%, Rutin, wobei die Angabe bezogen ist auf die Menge an eingesetztem Buchweizensamen. In absoluten Mengen enthält eine Gabeneinheit zumindest 0,1 g Rutin, vorzugsweise zumindest 0,5 g, insbesondere zumindest 1,0 g, wobei bezüglich der Bestimmung der Mengen oder Anteile auf den folgenden Absatz verwiesen wird. Zusätzlich enthalten können sein Lebensmittelzusatzstoffe, vorzugsweise ausgewählt aus der Gruppe bestehend aus Lebensmittelfarbstoffen, Konservierungsmitteln, Vitaminen und Mineralstoffen und/oder galenischen Hilfsstoffen. Lediglich beispielsweise kann die Zubereitung in lyophilisierter Variante in einer in der pharmazeutischen Industrie üblichen Kapsel verkapselt sein.

Die Erfindung betrifft auch eine Verwendung einer erfindungsgemäßen Zubereitung zur Herstellung eines funktionalen Lebensmittels, wobei dem Lebensmittel eine physiologisch wirksame Menge der Zubereitung zugegeben wird. Als physiologisch wirksame Menge ist beispielsweise eine Menge bezeichnet, welche zumindest 0,1 g Rutin, vorzugsweise zumindest 0,5 g, insbesondere zumindest 1,0 g, enthält. Dabei wird die Rutinmenge durch Messung des Gehaltes an Rutin in dem eingesetzten Buchweizen bestimmt. Eine beispielhafte Bestimmungsmethode ist in Gierlowska J., et al., Food/Nahrung Vol. 2, Issue 7, 705-709 (1958) beschrieben. Generell sind alle üblichen Verfahren mit Extraktion des Buchweizens mittels beispielsweise heißem Wasser oder Methanol und Analyse mit säulenchromatographischen Methoden einsetzbar. Die Erfindung umfasst auch ein in vorstehender Weise erhältliches funktionales Lebensmittel.

Weiterhin umfasst die Erfindung auch die Verwendung einer erfindungsgemäßen Zubereitung oder eines erfindungsgemäßen funktionalen Lebensmittels zur Herstellung einer Zusammensetzung zur Behandlung oder Prophylaxe einer Erkrankung aus der Gruppe bestehend aus periphere und autonome Neuropathien, zentralnervös degenerative Erkrankungen, Bluthochdruck, Atherosklerose, venöse Insuffizienz, Diabetes mellitus, Osteoporose, Katarakt und Photoaging der Haut.

Die Erfindung ist brauchbar für ein Verfahren zur Erhöhung der Bioverfügbarkeit eines Alkylcatechol, insbesondere von 4-MC und/oder von 3,4-Dihydroxyphenylessigsäure, in einem Organismus, insbesondere einem tierischen oder menschlichen Organismus, wobei eine erfindungsgemäße Zubereitung oder ein erfindungsgemäßes funktionales Lebensmittel dem Organismus in physiologisch wirksamer Dosis dargereicht wird, insbesondere oral bzw. zum Verzehr dargereicht wird.

Erfindungsgemäße Zubereitungen sind nicht nur im humanmedizinischen Bereich einsetzbar, sondern auch im veterinärmedizinischen Bereich. Dann wird es sich meist empfehlen, mit einer laktosefreien Variante der Erfindung zu arbeiten.

Im Folgenden wird die Erfindung anhand nicht limitierender Beispiele näher erläutert.

Nach oraler Gabe wird Rutin selbst nicht aus dem Darm in den Blutkreislauf aufgenommen. Rutin wird zunächst im Dickdarm durch die dortige Darmflora zu Quercetin-3-glucosid und Quercetin metabolisiert, das teilweise ins Blut übergeht bzw. in der Darm-Mucosa, aber auch in der Leber zu Quercetin-konjugaten umgewandelt wird. Der restliche Teil des Quercetins wird im Dickdarm von der Mikrobiota weiter verstoffwechselt. Die Hauptmetabolite sind 3,4-Dihydroxyphenylessigsäure sowie 4-MC.

Ein großer Teil der die Gesundheit fördernden Wirkungen des Rutins werden dem 4-MC zugeschrieben (siehe Einleitung). Es war daher erstrebenswert, die "Bioverfügbarkeit" von 4-MC verlässlich anzuheben.

Als Maß der "Bioverfügbarkeit" wurde die Gesamtausscheidung von 4-MC im Urin von gesunden Probanden herangezogen nach Einnahme von verschiedenen Zubereitungen von tartarischem Buchweizen.

Aus der Tabelle geht hervor, das nach Verzehr von warmem Brei aus 75 g gemahlenem Buchweizen, dem zuvor 500 ml kochendes Wasser zugesetzt worden war, die renale Gesamtausscheidung von 4-MC in einer Größenordnung liegt, die vergleichbar ist mit der 4-MC Gesamtausscheidung nach oraler Einnahme von 2 g Rutin in heißer Milch. (Die in 75 g gemahlenem Buchweizen enthaltene Menge an Rutin entspricht etwa 2 g Rutin).

Überraschenderweise lag die renale 4-MC Gesamtausscheidung nach Verzehr von warmem Brei aus 75 g gemahlenem Buchweizen, der zuvor 500 ml kochende Milch zugesetzt worden war, um das 3-fache über der Ausscheidung nach Verzehr der entsprechenden o.a. Zubereitung mit kochendem Wasser.

**Tabelle: Zubereitung von Buchweizen mit Milch: Renale Ausscheidung von 4-Methylcatechol (4-MC), Dihydroxyphenylessigsäure (DOPAC) und Quercetin von gesunden Probanden; Urin-Sammelperiode 0 bis 48 Std.**

| Zubereitung | 4- MC, mg Gesamtausscheidung | DOPAC, mg Gesamtausscheidung | Quercetin, mg Gesamtausscheidung |
|---|---|---|---|
| 75g tart. Buchweizen in 500 ml gekochtem Wasser | 7,33 | 14,72 | 3,33 |
| 40 g tart. Buchweizen in 400 ml kalter Milch | 5,43 | 5,16 | |
| (X 1,875 d.h. entsprechend 75 g Buchweizen) | (10,2) | (9,67) | |
| 75 g tart. Buchweizen in 400 ml kalter und dann aufgekochter Milch | 19,3 | 11,23 | 15,64 |
| 75 g tart. Buchweizen in 500 ml kochender Milch | 23,5 | 15,49 | 3,57 |
| Zum Vergleich: | | | |
| 2 g Rutin in 500 ml heißer Milch | 6,0 | 6,53 | |

## Patentansprüche

1. Verfahren zur Herstellung einer diätetischen oder pharmazeutischen Zubereitung mit den folgenden Verfahrensschritten:
a) gemahlener tartarischer Buchweizen, wird mit einer Proteinlösung, welche ausgewählt ist aus der Gruppe bestehend aus Kuhmilch, Buttermilch, Kamelmilch, Stutenmilch, Ziegenmilch, Eselsmilch, Lamamilch, Rentiermilch, Wasserbüffelmilch, Schafsmilch, Sojamilch, Kokosmilch und laktosefreie Milch der vorstehenden Milcharten, im Gewichtsverhältnis Proteinlösung zu Buchweizen von 4 bis 20 gemischt, wobei die Proteinlösung vor der Zugabe des Buchweizens auf eine Temperatur von zumindest 60 °C gebracht und bis zur Zugabe des Buchweizens auf dieser Temperatur gehalten wird,
b) die Mischung aus der Stufe a) wird für eine Dauer von zumindest 10 - 1000 s auf einer Temperatur von zumindest 80 °C gehalten, und
c) anschließend an Stufe b) wird die Mischung auf eine Temperatur unterhalb 45 °C abgekühlt.

2. Verfahren nach Anspruch 1, wobei die Proteinlösung einen Fettgehalt von 0,5 - 8%, insbesondere 1,3 - 6%, aufweist.

3. Verfahren nach einem der Ansprüche 1 oder 2, wobei die Proteinlösung unbehandelt, pasteurisiert, einem Extended Shelf Life (ESL) Verfahren unterworfen, oder ultrahocherhitzt ist.

4. Verfahren nach einem der Ansprüche 1 bis 3, wobei die Proteinlösung vor der Zugabe des Buchweizens auf eine Temperatur von zumindest 70 °C, insbesondere zumindest 80 °C, vorzugsweise zumindest 90 °C, gebracht und bis zur Zugabe des Buchweizens auf dieser Temperatur gehalten wird.

5. Verfahren nach einem der Ansprüche 1 bis 4, wobei in Stufe b) die Mischung auf eine Temperatur von zumindest 90 °C, gebracht und gehalten wird.

6. Verfahren nach einem der Ansprüche 1 bis 5, wobei in Stufe c) die Mischung auf eine Temperatur von 5 - 40°C, insbesondere 8 - 30 °C, abgekühlt wird.

7. Verfahren nach einem der Ansprüche 1 bis 6, wobei anschließend an Stufe c) eine Trocknung der Zubereitung durchgeführt wird.

8. Diätetische oder pharmazeutische Zubereitung erhältlich mit einem Verfahren nach einem der Ansprüche 1 bis 7.

9. Zubereitung nach Anspruch 8, enthaltend 0,5 - 10 Gew.-%, insbesondere 1 - 5 Gew.-%, vorzugsweise 2 - 4 Gew.-%, Rutin, wobei die Angabe bezogen ist auf die Menge an eingesetztem Buchweizen.

10. Zubereitung nach einem der Ansprüche 8 oder 9, zusätzlich enthaltend Lebensmittelzusatzstoffe, vorzugsweise ausgewählt aus der Gruppe bestehend aus Lebensmittelfarbstoffen, Konservierungsmitteln, Vitaminen und Mineralstoffen und/oder galenischen Hilfsstoffen.

11. Verwendung einer Zubereitung nach einem der Ansprüche 8 bis 10 zur Herstellung eines funktionalen Lebensmittels, wobei dem Lebensmittel eine physiologisch wirksame Menge der Zubereitung zugegeben wird.

12. Funktionales Lebensmittel erhältlich gemäß Anspruch 11.

13. Zubereitung nach einem der Ansprüche 8 bis 10 oder funktionales Lebensmittel nach Anspruch 12 zur Verwendung bei der Behandlung oder Prophylaxe einer Erkrankung von Mensch oder Tier aus der Gruppe bestehend aus periphere und autonome Neuropathien, zentralnervös degenerative Erkrankungen, Bluthochdruck, Atherosklerose, venöse Insuffizienz, Diabetes mellitus, Osteoporose Katarakt und Photoaging der Haut.

## Claims

1. A method for producing a dietetic or pharmaceutical preparation, comprising the following steps:
a) ground tartary buckwheat is mixed with a protein solution, which is selected from the group consisting of cow milk, buttermilk, camel milk, mare milk, goat milk, donkey milk, lama milk, reindeer milk, water buffalo milk, sheep milk, soy milk, coconut milk, and lactose-free milk of the above milk types, in a weight ratio of protein solution to buckwheat from 4 to 20, prior to the addition of the buckwheat the protein solution being heated to a temperature of at least 60 °C and being held on this temperature till the addition of the buckwheat,
b) the mixture of step a) is held for a time of at least 10 to 1000 s on a temperature of at least 80 °C, and
c) subsequently to step b), the mixture is cooled down to a temperature below 45 °C.

2. The method of claim 1, wherein the protein solution has a fat content from 0.5 to 8 %, in particular from 1.3 to 6 %.

3. The method of one of claims 1 or 2, wherein the protein solution is untreated, pasteurized, subjected to an Extended Shelf Life (ESL) method, or UHT treated.

4. The method of one of claims 1 to 3, wherein prior to the addition of the buckwheat the protein solution is heated to a temperature of at least 70 °C, in particular at least 80 °C, preferably at least 90 °C, and is held on this temperature till the addition of the buckwheat.

5. The method of one of claims 1 to 4, wherein in step b) the mixture is heated to a temperature of at least 90 °C, and is held on this temperature.

6. The method of one of claims 1 to 5, wherein in step c) the mixture is cooled down to a temperature from 5 to 40°C, in particular from 8 to 30 °C.

7. The method of one of claims 1 to 6, wherein subsequently to step c), a drying process of the preparation is carried out.

8. A dietetic or pharmaceutical preparation obtainable by a method of one of claims 1 to 7.

9. A preparation of claim 8, containing 0.5 to 10 wt.-%, in particular 1 to 5 wt.-%, preferably 2 to 4 wt.-%, of rutin, wherein the indicated value is referred to the amount of buckwheat employed.

10. The preparation of one of claims 8 or 9, in addition containing food additives, preferably selected from the group consisting of food colors, preservation agents, vitamins, and minerals and/or galenic excipients.

11. Use of a preparation of one of claims 8 to 10 for producing a functional food, wherein to the food is added a physiologically effective amount of the preparation.

12. A functional food obtainable according to claim 11.

13. The preparation of one of claims 8 to 10 or the functional food of claim 12 for use in the treatment or prophylaxis of a disease of man or animal from the group consisting of peripheral and autonomous neuropathies, central nervous degenerative diseases, hypertension, arteriosclerosis, venous insufficiency, diabetes mellitus, osteoporosis cataract, and photoaging of the skin.

## Revendications

1. Procédé de production d'une préparation diététique ou pharmaceutique, comprenant les étapes suivantes:
a) du sarrasin tartarique moulu est mélangé avec une solution de protéine, qui est choisie à partir du groupe consistant en du lait de vache, petit lait, lait de chameau, lait de jument, lait de chèvre, lait d'âne, lait de lama, lait de renne, lait de buffle d'eau, lait de brebis, lait de soja, lait de noix de coco, et lait sans lactose des laits ci-dessus, dans un rapport pondéral de solution de protéine à sarrasin entre 4 et 20, avant l'addition du sarrasin la solution de protéine étant chauffée à une température d'au moins 60 °C et étant maintenue à cette température jusqu'à l'addition du sarrasin,
b) le mélange de étape a) est maintenu pendant un temps d'au moins 10 à 1000 s à une température d'au moins 80 °C, et
c) après l'étape b) le mélange est refroidi à une température inférieure à 45 °C.

2. Procédé selon la revendication 1, dans lequel la solution de protéine a une teneur en matières grasses entre 0,5 et 8 %, en particulier entre 1,3 et 6 %.

3. Procédé selon une des revendications 1 ou 2, dans lequel la solution de protéine est non-traitée, pasteurisée, soumise à un procédé Extended Shelf Life (ESL), ou stérilisée UHT.

4. Procédé selon une des revendications 1 à 3, dans lequel avant l'addition du sarrasin la solution de protéine est chauffée à une température d'au moins 70 °C, en particulier au moins 80 °C, de préférence au moins 90 °C, et est maintenue à cette température jusqu'à l'addition du sarrasin.

5. Procédé selon une des revendications 1 à 4, dans lequel dans l'étape b) le mélange est chauffé à une température d'au moins 90 °C, et est maintenu à cette température.

6. Procédé selon une des revendications 1 à 5, dans lequel dans l'étape c) le mélange est refroidi à une température entre 5 et 40°C, en particulier entre 8 et 30 °C.

7. Procédé selon une des revendications 1 à 6, dans lequel après l'étape c), un procédé de séchage de la préparation est effectué.

8. Préparation diététique ou pharmaceutique à être obtenue par un procédé selon une des revendications 1 à 7.

9. Préparation selon la revendication 8, comportant entre 0,5 et 10 % en poids, en particulier entre 1 et 5 % en poids, de préférence entre 2 et 4 % en poids, de la rutine, la valeur étant rapportée à la quantité de sarrasin utilisé.

10. Préparation selon une des revendications 8 ou 9, en addition comportant des additifs alimentaires, de préférence choisis à partir du groupe consistant en colorants, agents conservateurs, vitamines, et éléments minéraux et/ou excipients galéniques.

11. Utilisation d'une préparation selon une des revendications 8 à 10 pour la production d'un produit alimentaire fonctionnel, au produit alimentaire étant ajoutée une quantité physiologiquement efficace de la préparation.

12. Produit alimentaire fonctionnel à être obtenu selon la revendication 11.

13. Préparation selon une des revendications 8 à 10 ou produit alimentaire fonctionnel selon la revendication 12 pour l'utilisation dans le traitement ou la prophylaxie d'une maladie humaine ou animale à partir du groupe consistant en des neuropathies périphériques et autonomes, des maladies dégénératives nerveuses centrales, de l'hypertension, de l'artériosclérose, de l'insuffisance veineuse, du diabète sucré, de la cataracte d'ostéoporose, et du photo-vieillissement de la peau.
